# EUROPEAN PATENT APPLICATION

(11) **EP 4 733 411 A1**
(43) Date of publication of application: **29.04.2026**
(21) Application number: 24825989.7
(22) Date of filing: 20.06.2024
(51) Int. Cl.: C12Q 1/686, C07K 14/195, C12N 9/12, C12N 15/31

(54) **METHOD FOR REDUCING AMPLIFICATION BIAS IN NUCLEIC ACID AMPLIFICATION**

(30) Priority: 23.06.2023 JP 2023103775
(71) Applicant: Toyobo Co., Ltd., Kita-ku Osaka-shi, Osaka 530-0001 (JP)
(72) Inventor: KOBAYASHI, Tetsuhiro, Osaka-shi, Osaka 530-0001 (JP)
(74) Representative: Vossius & Partner Patentanwälte Rechtsanwälte mbB
(86) International application number: PCT/JP2024/022454
(87) International publication number: WO 2024/262588

(57) **Abstract**

An object of the present invention is to provide a method for reducing amplification bias in multiplex PCR. In the present invention, amplification bias was found to be reduced by amplifying nucleic acids by using a proliferating cell nuclear antigen. The present invention relates to a method for reducing amplification bias by using a proliferating cell nuclear antigen, as well as to a reagent and a kit for use in the method.

## Description

### Technical Field

The present invention relates to a method for reducing amplification bias in nucleic acid amplification and other related aspects. The present invention can be used not only in research fields but also in clinical diagnostics and other applications.

### Background Art

Polymerase chain reaction (PCR) is a method for amplifying target nucleic acids in a sample by repeating a cycle consisting of three steps: (1) DNA denaturation (dissociation of double-stranded DNA into single-stranded DNA) by heat treatment, (2) annealing of primers to single-stranded template DNA, and (3) extension of the primers using DNA polymerases.

PCR can amplify target nucleic acids several hundred thousand times even from a minute sample containing only a few copies, and it has come to be widely used not only in research fields but also in forensic fields such as genetic diagnosis and clinical diagnosis, as well as in microbial testing of food and environmental samples.

In PCR reactions, multiple target nucleic acids can be simultaneously amplified by using multiple primers or by identifying homologous regions among the target nucleic acids and designing primers that correspond to these regions. This type of PCR that amplifies multiple target nucleic acids in a single reaction is called "multiplex PCR." Multiplex PCR is used not only in research, but also in diagnostics of infectious disease and other fields due to its ability to detect multiple target nucleic acids concurrently. With the recent growing use of next-generation sequencing (NGS), multiplex PCR has been increasingly applied to library amplification in sequencing processes.

Multiplex PCR is expected to amplify all regions uniformly. However, due to factors such as PCR reaction conditions and DNA sequence-dependent biases, certain regions may be amplified more efficiently than others, resulting in amplification bias. When amplification bias occurs, only regions that are readily amplified are over-detected, while the detection of other regions is decreased, potentially leading to the oversight of these other regions. Particularly in NGS, this amplification bias reduces the reliability of quantitative measurements and degrades sequencing precision. Moreover, significant amplification bias can pose a challenge, requiring a greater number of sequencing reads to confirm the entire gene sequence, which substantially increases costs.

In fact, to reduce amplification bias during NGS library preparation, studies on PCR reaction conditions or additives have been conducted (NPL 1). However, the performance is not yet sufficient for practical use, and further improvements are required.

### Citation List

### Non-patent Literature

NPL 1: Genome Biology, Vol. 12, Article Number: R18 (2011), Analyzing and minimizing PCR amplification bias in Illumina sequencing libraries, Daniel Aird et al.

### Summary of Invention

### Technical Problem

An object of the present invention is to provide a method for reducing amplification bias in multiplex PCR, and other related methods. Another object of the present invention is to provide a reagent and a kit suitable for this purpose.

### Solution to Problem

The method and other aspects of the present invention for achieving the objects above include a method for reducing amplification bias in multiplex PCR, wherein a proliferating cell nuclear antigen is used in nucleic acid amplification. Specifically, the present inventor found that using a proliferating cell nuclear antigen in multiplex PCR reduces amplification bias, and the inventor completed the invention.

The major embodiments of the present invention include the following.

### Item 1.

A method for reducing amplification bias in multiplex PCR, comprising the step of amplifying nucleic acids in a reaction solution containing a proliferating cell nuclear antigen.

### Item 2.

The method according to Item 1, wherein the proliferating cell nuclear antigen comprises
(a) a polypeptide containing an amino acid sequence in which at least one amino acid selected from the group consisting of amino acids at positions 82, 84, and 109 in the amino acid sequence of SEQ ID NO: 1 or 2 is mutated, or
(b) a polypeptide containing an amino acid sequence in which at least one amino acid selected from the group consisting of amino acids at positions 139, 143, and 147 in the amino acid sequence of SEQ ID NO: 1 or 2 is mutated, or
(c) a polypeptide containing an amino acid sequence in which at least one amino acid selected from the group consisting of amino acids at positions 82, 84, and 109 in the amino acid sequence of SEQ ID NO: 1 or 2, and at least one amino acid selected from the group consisting of amino acids at positions 139, 143, and 147 in the amino acid sequence of SEQ ID NO: 1 or 2 are mutated, or
(d) a polypeptide containing an amino acid sequence in which one to several amino acids are mutated among amino acids other than amino acids corresponding to positions 82, 84, 109, 139, 143, and 147 in the amino acid sequence of SEQ ID NO: 1 or 2 in any of the amino acid sequences contained in the polypeptides (a) to (c), or
(e) a polypeptide containing an amino acid sequence having at least 80% identity with any of the amino acid sequences contained in the polypeptides (a) to (c), wherein at least one amino acid corresponding to a position selected from the group consisting of positions 82, 84, 109, 139, 143, and 147 in the amino acid sequence of SEQ ID NO: 1 or 2 is mutated.

### Item 3.

The method according to Item 2,
wherein
the amino acid corresponding to position 143 is a basic amino acid,
the amino acid corresponding to position 147 is a neutral amino acid, or
the amino acid corresponding to position 143 is a basic amino acid and the amino acid corresponding to position 147 is a neutral amino acid.

### Item 4.

The method according to Item 2 or 3,
wherein
the amino acid corresponding to position 143 is arginine or lysine,
the amino acid corresponding to position 147 is alanine or glycine, or
the amino acid corresponding to position 143 is arginine or lysine and the amino acid corresponding to position 147 is alanine or glycine.

### Item 5.

The method according to any one of Items 2 to 4, wherein in the amino acid sequence contained in any of the polypeptides (a) to (e), the amino acid corresponding to position 73 in the amino acid sequence of SEQ ID NO: 1 or 2 is leucine. Item 6.

The method according to any one of Items 2 to 5, wherein the polypeptide (d) contains an amino acid sequence in which one to three amino acids are mutated among amino acids other than amino acids corresponding to positions 82, 84, 109, 139, 143, and 147 in the amino acid sequence of SEQ ID NO: 1 or 2 in any of the amino acid sequences contained in the polypeptides (a) to (c).

### Item 7.

The method according to any one of Items 2 to 6, wherein the mutated means that an amino acid is substituted, deleted, inserted, and/or added.

### Item 8.

The method according to any one of Items 2 to 7, wherein the polypeptide (e) contains an amino acid sequence having at least 90% identity with any of the amino acid sequences contained in the polypeptides (a) to (c), wherein at least one amino acid corresponding to a position selected from the group consisting of positions 82, 84, 109, 139, 143, and 147 in the amino acid sequence of SEQ ID NO: 1 or 2 is mutated.

### Item 9.

The method according to any one of Items 1 to 8, wherein at least one nucleic acid amplified in the multiplex PCR has a GC content of 80% or higher.

### Item 10.

The method according to any one of Items 1 to 9, wherein the reaction solution further contains a DNA polymerase belonging to family B.

### Item 11.

The method according to Item 10, wherein the DNA polymerase belonging to family B is derived from Archaea.

### Item 12.

The method according to Item 10, wherein the DNA polymerase belonging to family B is derived from bacteria of the genus *Pyrococcus* or the genus *Thermococcus.*

### Item 13.

A method for improving amplification bias in multiplex PCR for preparation of a library used in next-generation sequencing, comprising the step of amplifying nucleic acids in a reaction solution containing a proliferating cell nuclear antigen.

### Item 14.

A method for preparing a library for use in next-generation sequencing, comprising the step of performing multiplex PCR in a reaction solution containing a proliferating cell nuclear antigen.

### Item 15.

A method for improving amplification of a target nucleic acid having a GC content of 80% or higher in multiplex PCR, comprising the step of amplifying nucleic acids in a reaction solution containing a proliferating cell nuclear antigen.

### Item 16.

A multiplex PCR comprising the step of amplifying a nucleic acid having a GC content of 80% or higher in a reaction solution containing a proliferating cell nuclear antigen.

### Item 17.

A reagent for performing the method of any one of Items 1 to 15 or performing the multiplex PCR of Item 16, comprising a proliferating cell nuclear antigen containing
(a) a polypeptide containing an amino acid sequence in which at least one amino acid selected from the group consisting of amino acids at positions 82, 84, and 109 in the amino acid sequence of SEQ ID NO: 1 or 2 is mutated, or
(b) a polypeptide containing an amino acid sequence in which at least one amino acid selected from the group consisting of amino acids at positions 139, 143, and 147 in the amino acid sequence of SEQ ID NO: 1 or 2 is mutated, or
(c) a polypeptide containing an amino acid sequence in which at least one amino acid selected from the group consisting of amino acids at positions 82, 84, and 109 in the amino acid sequence of SEQ ID NO: 1 or 2, and at least one amino acid selected from the group consisting of amino acids at positions 139, 143, and 147 in the amino acid sequence of SEQ ID NO: 1 or 2 are mutated, or
(d) a polypeptide containing an amino acid sequence in which one to several amino acids are mutated among amino acids other than amino acids corresponding to positions 82, 84, 109, 139, 143, and 147 in the amino acid sequence of SEQ ID NO: 1 or 2 in any of the amino acid sequences contained in the polypeptides (a) to (c), or
(e) a polypeptide containing an amino acid sequence having at least 80% identity with any of the amino acid sequences contained in the polypeptides (a) to (c), wherein at least one amino acid corresponding to a position selected from the group consisting of positions 82, 84, 109, 139, 143, and 147 in the amino acid sequence of SEQ ID NO: 1 or 2 is mutated.

### Item 18.

A kit for performing the method of any one of Items 1 to 15 or performing the multiplex PCR of Item 16, comprising the reagent of Item 17.

### Advantageous Effects of Invention

The present invention reduces amplification bias in multiplex PCR. Thus, the present invention reduces the likelihood of overlooking regions that are difficult to amplify, enabling more accurate amplification. Additionally, the present invention improves sequencing accuracy and cost-effectiveness in NGS.

### Brief Description of Drawings

Fig. 1 shows the normalized coverage of KOD (with and without a PCNA).
Fig. 2 shows the normalized coverage of KOD (with and without a PCNA).
Fig. 3 shows the normalized coverage of PS (with and without a PCNA).
Fig. 4 shows the alignment of the amino acid sequences of various PCNAs.

### Description of Embodiments

The present invention is described in more detail below with reference to embodiments of the invention.

In the present specification, abbreviated symbols using alphabetic notation may be used to represent base sequences, amino acid sequences, and their individual components. All such abbreviations and notations conform to the conventions established in the fields of molecular biology and genetic engineering. In the present specification, mutations in amino acid sequences are concisely indicated using notations such as "D143A." This notation (D143A) indicates that the aspartic acid at positon 143 has been substituted with alanine, denoting the original amino, its position, and the amino acid after substitution. The sequence numbers mentioned in the present specification correspond to the sequence numbers listed in the sequence listing, unless otherwise specified. Mutants with multiple mutations are indicated by using connected notations described above with a slash ("/"). For example, "D143A/D147A" indicates that the aspartic acid at position 143 has been substituted with alanine, and the aspartic acid at position 147 has been substituted with alanine.

In the present specification, the term "mutant" in "mutant PCNA" means that the PCNA has an amino acid sequence different from that of conventionally known PCNAs, and does not distinguish between mutations arising from artificial modification and those resulting from natural variation.

In the present specification, the phrase "corresponding to" in the expression "amino acids corresponding to positions ..." indicates that amino acids in a proliferating cell nuclear antigen containing an amino acid sequence that is not completely identical to the amino acid sequence of SEQ ID NO: 1 or 2 correspond to those at indicated positions in SEQ ID NO: 1 or 2 when the amino acid sequence in question is aligned with the amino acid sequence of SEQ ID NO: 1 or 2.

### (1) Method for Reducing Amplification Bias of the Present Invention

The method for reducing amplification bias according to the present invention is a method for reducing amplification bias in multiplex PCR, and a characteristic of the method is a proliferating cell nuclear antigen (PCNA) contained in a reaction solution. The phrase "reducing amplification bias" as used here encompasses the meaning that amplification bias in multiplex PCR using a reaction solution containing a PCNA is lower than that in multiplex PCR using a reaction solution without a PCNA.

### (2) Multiplex PCR

In the methods and other aspects of the present invention (in the present specification, the phrase "methods and other aspects of the present invention" is used to encompass the methods, multiplex PCR, reagent, and kit described in Items 1 to 18); "multiplex PCR" refers to PCR that amplifies multiple target nucleic acids in a single reaction. Multiplex PCR may use multiple primers to simultaneously amplify multiple target nucleic acids, or multiplex PCR may involve designing primers corresponding to a homologous region between multiple target nucleic acids and amplifying multiple target nucleic acids with a single primer pair. PCR involving ligating multiple gene fragments with adapters and using primers that hybridize to adapter regions (e.g., NGS library amplification) also falls within the scope of multiplex PCR because it can simultaneously amplify multiple target nucleic acids. The multiplex PCR used herein is not limited to these examples of multiplex PCR techniques.

### (3) PCNA

The proliferating cell nuclear antigen (or "PCNA" in the present specification) used in the methods and other aspects of the present invention is a type of PCR enhancing factor. The PCNA is preferably a PCNA that loads onto DNA independently. It is desirable for the PCNA to have heat resistance capable of withstanding the thermal cycles of PCR; preferably, the PCNA retains activity even after PCR. It is more preferred that the activity remains even after heat treatment at 80°C for 30 minutes, with the remaining activity being 50% or more, more preferably 70% or more, and even more preferably 90% or more. The PCNA may be a known wild-type PCNA or a mutant of a known wild-type PCNA.

PCNAs typically form a multimer and takes a ring-shaped structure. The phrase "loads onto DNA" refers to the process of DNA threading through the interior of the ring-shaped structure formed by a PCNA multimer. Typically, PCNAs can be loaded onto DNA only in conjunction with a factor called "replication factor C" (RFC). "A PCNA that loads onto DNA independently" refers to a wild-type or mutant PCNA that possesses the property of allowing DNA to easily thread through the ring-shaped structure of the PCNA multimer without RFC by mutating the sites involved in PCNA multimerization to destabilize the multimer formation, for example. The PCNA that can be used in the methods and other aspects of the present invention is preferably one that can load onto DNA without RFC and that facilitates the extension reaction of a DNA polymerase.

The PCNA used in the methods and other aspects of the present invention is more preferably PCNAs derived from bacteria of the genus *Pyrococcus* and the genus *Thermococcus.* PCNAs derived from the genus *Pyrococcus* include, but are not limited to, those isolated from *Pyrococcus furiosus* (SEQ ID NO: 2), *Pyrococcus sp.* GB-D, *Pyrococcus woesei, Pyrococcus abyssi,* and *Pyrococcus horikoshii.* PCNAs derived from the genus *Thermococcus* include, but are not limited to, those isolated from *Thermococcus kodakaraensis* (SEQ ID NO: 1), *Thermococcus gorgonarius, Thermococcus litoralis, Thermococcus sp.* JDF-3, *Thermococcus sp.* 9 degrees North-7 (*Thermococcus sp.* 9°N-7), *Thermococcus sp.* KS-*1, Thermococcus celer,* and *Thermococcus siculi.*

PCNA preferably comprises
(a) a polypeptide containing an amino acid sequence in which at least one amino acid selected from the group consisting of amino acids at positions 82, 84, and 109 in the amino acid sequence of SEQ ID NO: 1 or 2 is mutated (which is also referred to as "polypeptide A" below), or
(b) a polypeptide containing an amino acid sequence in which at least one amino acid selected from the group consisting of amino acids at positions 139, 143, and 147 in the amino acid sequence of SEQ ID NO: 1 or 2 is mutated (which is also referred to as "polypeptide B" below), or
(c) a polypeptide containing an amino acid sequence in which at least one amino acid selected from the group consisting of amino acids at positions 82, 84, and 109 in the amino acid sequence of SEQ ID NO: 1 or 2, and at least one amino acid selected from the group consisting of amino acids at positions 139, 143, and 147 in the amino acid sequence of SEQ ID NO: 1 or 2 are mutated (which is also referred to as "polypeptide C" below), or
(d) a polypeptide containing an amino acid sequence in which one to several amino acids are mutated among amino acids other than amino acids corresponding to positions 82, 84, 109, 139, 143, and 147 in the amino acid sequence of SEQ ID NO: 1 or 2 in any of the amino acid sequences contained in the polypeptides (a) to (c), or
(e) a polypeptide containing an amino acid sequence having at least 80% identity with any of the amino acid sequences contained in the polypeptides (a) to (c), wherein at least one amino acid corresponding to a position selected from the group consisting of positions 82, 84, 109, 139, 143, and 147 in the amino acid sequence of SEQ ID NO: 1 or 2 is mutated.

The amino acid at position 82 in the amino acid sequence of SEQ ID NO: 1 or 2 is arginine. The amino acids that substitute the arginine at position 82 include cysteine, alanine, proline, glycine, isoleucine, leucine, methionine, valine, phenylalanine, tryptophan, tyrosine, asparagine, glutamine, serine, threonine, aspartic acid, glutamic acid, histidine, and lysine, preferably cysteine, alanine, proline, glycine, isoleucine, leucine, methionine, valine, phenylalanine, tryptophan, tyrosine, asparagine, glutamine, serine, threonine, aspartic acid, and glutamic acid, and more preferably cysteine, alanine, proline, glycine, isoleucine, leucine, methionine, valine, phenylalanine, tryptophan, tyrosine, asparagine, glutamine, serine, and threonine.

The amino acid at position 84 in the amino acid sequence of SEQ ID NO: 1 or 2 is lysine. The amino acids that substitute the lysine at position 84 include alanine, proline, glycine, isoleucine, leucine, methionine, valine, phenylalanine, tryptophan, tyrosine, cysteine, asparagine, glutamine, serine, threonine, aspartic acid, glutamic acid, arginine, and histidine, preferably alanine, proline, glycine, isoleucine, leucine, methionine, valine, phenylalanine, tryptophan, tyrosine, cysteine, asparagine, glutamine, serine, threonine, aspartic acid, and glutamic acid, and more preferably alanine, proline, glycine, isoleucine, leucine, methionine, valine, phenylalanine, tryptophan, tyrosine, cysteine, asparagine, glutamine, serine, and threonine.

The amino acid at position 109 in the amino acid sequence of SEQ ID NO: 1 or 2 is arginine. The amino acids that substitute the arginine at position 109 include glutamic acid, alanine, aspartic acid, proline, glycine, leucine, isoleucine, methionine, valine, phenylalanine, tryptophan, tyrosine, cysteine, asparagine, glutamine, serine, threonine, histidine, and lysine, and preferably glutamic acid, alanine, aspartic acid, proline, glycine, leucine, isoleucine, methionine, valine, phenylalanine, tryptophan, tyrosine, cysteine, asparagine, glutamine, serine, and threonine.

The amino acid at position 139 in the amino acid sequence of SEQ ID NO: 1 or 2 is glutamic acid. The amino acids that substitute the glutamic acid at position 139 include alanine, proline, glycine, isoleucine, leucine, methionine, valine, phenylalanine, tryptophan, tyrosine, cysteine, asparagine, glutamine, serine, threonine, arginine, histidine, lysine, and aspartic acid, preferably alanine, proline, glycine, isoleucine, leucine, methionine, valine, phenylalanine, tryptophan, tyrosine, cysteine, asparagine, glutamine, serine, threonine, arginine, histidine, and lysine, and more preferably alanine, proline, glycine, isoleucine, leucine, methionine, valine, phenylalanine, tryptophan, tyrosine, cysteine, asparagine, glutamine, serine, and threonine.

The amino acid at position 143 in the amino acid sequence of SEQ ID NO: 1 is glutamic acid. The amino acids that substitute the glutamic acid at position 143 include arginine, lysine, histidine, alanine, proline, glycine, isoleucine, leucine, methionine, valine, phenylalanine, tryptophan, tyrosine, cysteine, asparagine, glutamine, serine, threonine, and aspartic acid. For example, basic amino acids such as arginine, lysine, and histidine, or alanine is preferred.

The amino acid at position 143 in the amino acid sequence of SEQ ID NO: 2 is aspartic acid. The amino acids that substitute the aspartic acid at position 143 include arginine, lysine, histidine, alanine, proline, glycine, isoleucine, leucine, methionine, valine, phenylalanine, tryptophan, tyrosine, cysteine, asparagine, glutamine, serine, threonine, and glutamic acid. For example, arginine, lysine, histidine, or alanine is preferred, with arginine or lysine being more preferred.

The amino acid at position 147 in the amino acid sequence of SEQ ID NO: 1 or 2 is aspartic acid. The amino acids that substitute the aspartic acid at position 147 include alanine, glycine, proline, isoleucine, leucine, methionine, valine, phenylalanine, tryptophan, tyrosine, cysteine, asparagine, glutamine, serine, threonine, arginine, histidine, lysine, and glutamic acid. For example, alanine, glycine, proline, isoleucine, leucine, methionine, valine, phenylalanine, tryptophan, tyrosine, cysteine, asparagine, glutamine, serine, threonine, arginine, histidine, or lysine is preferred. Alanine, glycine, valine, leucine, isoleucine, or histidine is more preferred. Alanine or glycine is even more preferred.

More preferably, the PCNA includes those in which the amino acid corresponding to position 143 in the amino acid sequence of SEQ ID NO: 1 is mutated to a basic amino acid, and those in which the amino acid corresponding to position 147 in the amino acid sequence of SEQ ID NO: 1 is mutated to a neutral amino acid. As for neutral amino acids, natural ones include glycine, alanine, valine, leucine, isoleucine, phenylalanine, tyrosine, tryptophan, proline, serine, threonine, cysteine, methionine, asparagine, and glutamine. Glycine, alanine, valine, leucine, and isoleucine are preferred. Glycine and alanine are more preferred. Alanine, which has the least impact on the three-dimensional structure of the surrounding sites of the substitution site, is most preferred. As for basic amino acids, natural ones include arginine, histidine, and lysine. Preferred basic amino acids are arginine and lysine, and more preferred is arginine.

The sites involved in PCNA multimer formation include the amino acids at positions 82, 84, and 109 in the N-terminal region and the amino acids at positions 139, 143, and 147 in the C-terminal region (these six amino acids may also be referred to below as "the amino acids at the predetermined positions") in a PCNA derived from *Thermococcus kodakaraensis* (SEQ ID NO: 1) and a PCNA from *Pyrococcus furiosus* (SEQ ID NO: 2). It is believed that the N-terminal region is positively charged, whereas the C-terminal region is negatively charged, and that these regions interact each other to form a multimer.

The PCNA may be a polypeptide comprising an amino acid sequence in which one to several amino acids are substituted, deleted, inserted, and/or added (which are collectively referred to as "mutation") in any one of the amino acid sequences of the polypeptides A to C as long as mutations of the amino acids corresponding to the predetermined positions are conserved and the PCNA is preferably one that loads onto DNA independently. The number of mutated amino acids can be 1 to 49, 1 to 24, 1 to 12, 1 to 11, 1 to 10, 1 to 9, 1 to 8, 1 to 7, 1 to 6, 1 to 5, etc., with 1 to 4 being preferred, and 1 to 3 being more preferred. The "one to several mutations" can be made by introducing mutations into DNA encoding the PCNA of the present invention using a known technique, such as restriction enzyme treatment, processing with exonucleases or DNA ligases, sitedirected mutagenesis, or random mutagenesis (Molecular Cloning, Third Edition, Chapter 13, Cold Spring Harbor Laboratory Press, New York). Furthermore, variant PCNA monomers can also be obtained according to other methods, such as ultraviolet irradiation. Variant PCNA monomers include naturally occurring variants based on individual differences in microorganisms that retain PCNAs, as well as differences in species or genera (e.g., single-nucleotide polymorphisms).

The PCNA may be a polypeptide comprising an amino acid sequence having at least 80% identity compared to the amino acid sequence of any one of the polypeptides A to C provided that mutations of the amino acids corresponding to the predetermined positions are conserved. It is preferred that the PCNA can load onto DNA independently. Preferably, the identity between the amino acid sequence of the PCNA and the amino acid sequence of any one of the polypeptides A to C is at least 85%, more preferably at least 88%, still more preferably at least 90%, even more preferably at least 93%, yet more preferably at least 95%, particularly preferably at least 98%, and most preferably at least 99%. Such polypeptides comprising an amino acid sequence having a certain degree of identity or higher can be prepared based on the known genetic engineering techniques described above.

The proliferating cell nuclear antigen is particularly preferably composed of a polypeptide containing an amino acid sequence represented by SEQ ID NO: 1, 2, 9, 11, 13, 15, 17, 19, 21, 23, 25, or 27.

Various methods for calculating the identity of amino acid sequences are known. For example, the identity of amino acid sequences can be calculated using commercially available analysis tools or those accessible via telecommunications networks (the internet). In the present specification, the identity of amino acid sequences is calculated using the default parameters (initial settings) in the homology algorithm BLAST (basic local alignment search tool) of the National Center for Biotechnology Information (NCBI) at http://www.ncbi.nlm.nih.gov/BLAST/.

The PCNA used in the methods and other aspects of the present invention may be composed of a polypeptide containing an amino acid sequence in which the methionine corresponding to position 73 in the amino acid sequence of SEQ ID NO: 1 or SEQ ID NO: 2 has been mutated in order to increase the expression level. More preferably, the PCNA used in the methods and other aspects of the present invention may be, but is not limited to, a PCNA composed of a polypeptide containing an amino acid sequence with mutation M73L.

More specifically, the PCNA that can be used in the methods and other aspects of the present invention may be, but is not limited to, PCNA mutants described in WO2007/004654 or Japanese Patent No. 6741061, as well as PCNAs composed of polypeptides containing a sequence in which the amino acid corresponding to position 147 in the amino acid sequence of SEQ ID NO: 1 or 2 is substituted with alanine or glycine (D147A, D147G), a sequence in which the amino acid corresponding to position 143 is substituted with arginine or lysine (E143R, E143K, or D143R, D143K), a sequence in which the amino acids corresponding to positions 82 and 143 are both changed to alanine (R82A/E143A or R82A/D143A), a sequence in which the amino acid residues corresponding to positions 109 and 143 are both changed to alanine (R109A/E143A or R109A/D143A), and so on.

In a preferred embodiment, the amino acid sequence contained in the polypeptide constituting the PCNA may have one or more amino acid mutations selected from the group consisting of M73L, R82C, R82A, R109E, R109A, E139R, E139A, D143R, D143A, D143H, D143K, E143R, E143A, E143H, E143K, D147R, and D147A, and preferably one or more amino acid mutations selected from the group consisting of M73L, D143A, D143A/D147A, D143R, R109A/D143A, R82A/D143A, and D147A.

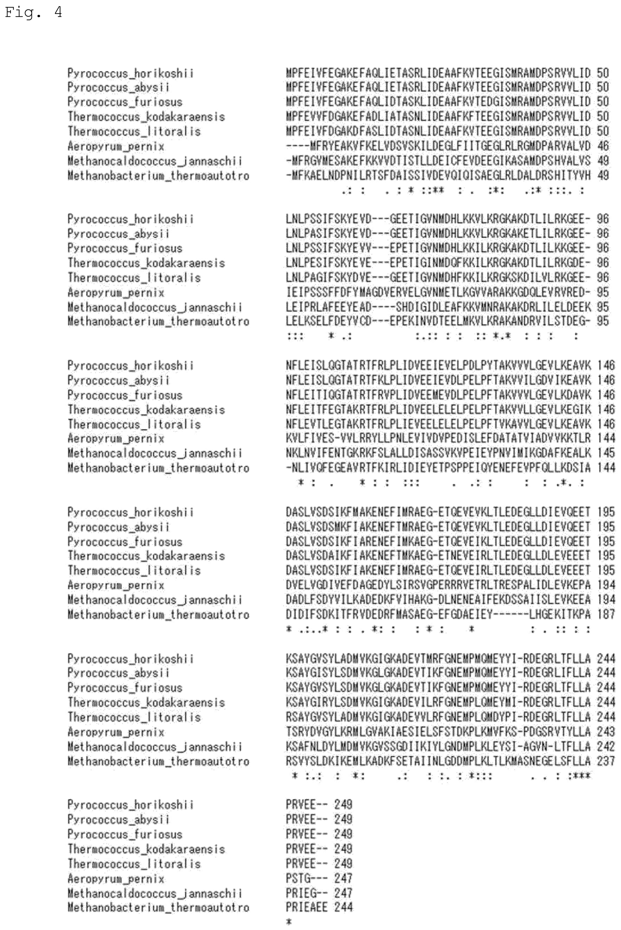

The details provided above and below using the amino acid sequence of SEQ ID NO: 1 or SEQ ID NO: 2 as examples are also applicable to PCNAs other than the PCNAs presented with specific sequences in the present specification. For example, as shown in Fig. 4, the mutation positions in PCNAs other than the PCNAs composed of polypeptides containing the amino acid sequence of SEQ ID NO: 1 or 2 are the positions of amino acids corresponding to amino acids involved in multimer formation, such as the amino acids at positions 82, 84, 109, 139, 143, and 147 in the amino acid sequence of SEQ ID NO: 1.

The method for producing the PCNA that can be used in the methods and other aspects of the present invention is not particularly limited; the PCNA can be produced according to a method known to those skilled in the art. For example, DNA having a base sequence encoding the amino acid sequence of the polypeptide as described above can be incorporated into a vector, which is then expressed in cells such as *Escherichia coli,* optionally followed by purifying and isolating the obtained PCNA according to a standard method of protein purification to produce a desired PCNA. Such DNA includes DNA that differs due to codon degeneracy.

The DNA above preferably has a base sequence with an identity of at least 80%, preferably at least 85%, more preferably at least 88%, even more preferably at least 90%, still more preferably at least 93%, yet more preferably at least 95%, particularly preferably at least 98%, and most preferably at least 99% to the base sequence represented by SEQ ID NO: 3 or 4, as long as the mutations of the amino acids corresponding to the predetermined positions in the protein encoded by the DNA are conserved. It is preferred that the protein encoded by the base sequence is a PCNA that loads onto DNA independently.

Various methods for calculating the identity of base sequences are known. For example, the identity of base sequences can be calculated using commercially available analysis tools or those accessible via telecommunications networks (e.g., the internet). In the present specification, the identity (%) of nucleotide sequences is calculated by conducting a search using the Advanced BLAST 2.1 homology algorithm of the National Center for Biotechnology Information (NCBI) with the BLASTN program and parameters set to the default values.

The DNA above may also hybridize under stringent conditions to a base sequence complementary to the base sequence represented by SEQ ID NO: 3 or 4 as long as the mutations of amino acids corresponding to the predetermined positions in the protein encoded by the DNA are conserved; it is preferred that the protein encoded by the base sequence be a PCNA that loads onto DNA independently.

The vector can be selected appropriately taking into consideration the type of host cell. Examples include plasmid vectors, cosmid vectors, phage vectors, and viral vectors (e.g., adenovirus vectors, adeno-associated virus vectors, retrovirus vectors, and herpesvirus vectors). Additionally, vectors suitable for filamentous fungi as hosts or vectors suitable for selfcloning are also usable.

The host cell is not particularly limited as long as it is capable of expressing DNA encoding a PCNA to produce the PCNA. Specifically, prokaryotic cells, such as *Escherichia coli* and *Bacillus subtilis,* and eukaryotic cells, such as yeast, mold, insect cells, plant culture cells, and mammalian cells, are usable.

While any method may be used to purify the PCNA expressed by host cells, examples include the following methods. After the host cells obtained by culture in a nutrient medium are harvested, they are disrupted and extracted by an enzymatic or physical crushing method to obtain a crude enzyme solution. The crude enzyme extract obtained is subjected to heat treatment, for example, at 80°C for 30 minutes, followed by ammonium sulfate precipitation to recover the PCNA fraction. This crude enzyme solution can be desalted according to a method such as gel filtration using Sephadex G-25 (manufactured by Amersham Pharmacia Biotech, Inc.). After this operation, the desalted solution is purified by Q Sepharose column chromatography to obtain a purified enzyme preparation. The purified enzyme preparation is refined by SDS-PAGE to a degree that it shows a substantially single band.

When obtaining a PCNA, it is preferable to mutate the methionine corresponding to position 73 in the amino acid sequence of SEQ ID NO: 1 or 2 to leucine (M73L) in order to increase the expression level of the PCNA. When a PCNA is prepared as a recombinant protein using *E. coli* as a host, a protein of about 20 kDa starting from the 73rd residue at the N-terminus may be generated as a by-product in addition to a translated protein starting from the original, initial Met. However, M73L mutation can suppress the production of such protein of about 20 kDa, and the PCNA (M73L) thus prepared exhibits properties indistinguishable from those of normal PCNAs. Therefore, the protein starting from M73L also falls within the definition of the PCNA as referred to in the present invention.

The PCNA may have various modifications. For example, a PCNA composed of a recombinant protein with any desired peptide or protein attached can be obtained by inserting DNA encoding the PCNA and other appropriate DNAs into the same vector, and using the vector to produce the recombinant protein. Additionally, modifications, such as the addition of glycans and/or lipids, or modifications that cause N-terminal or C-terminal processing, may be made. Such modifications can simplify the extraction and purification of recombinant proteins, or enable the addition of biological functions, for example.

PCNAs can form multimers through the interface created by the N-terminal region of one monomer joining with the C-terminal region of another monomer. Because this joining is reversible, at least a portion of the PCNA expressed as monomers may form a multimer.

Whether a PCNA can load onto DNA independently can be evaluated by PCR. For example, the ability to load onto DNA independently can be confirmed by adding the target PCNA to a standard PCR reaction solution containing template DNA, a buffer, magnesium, dNTPs, primers, and a DNA polymerase, and comparing its amplification amount with that of a solution without a PCNA and with that of a solution containing a PCNA known not to load onto DNA independently. The PCNA that does not load onto DNA independently, when added, does not change the amplification amount in PCR, and instead tends to reduce the amplification amount. On the other hand, mutants that can independently load onto DNA can achieve excellent amplification amounts as compared to a solution without a PCNA and a solution containing a PCNA known not to load onto DNA independently.

In the present invention, the evaluation of whether a PCNA can load onto DNA independently (evaluation of amplification enhancement activity) is conducted according to the following method.

250 ng of a PCNA to be evaluated is added to 50 µl of a reaction solution containing 1× PCR Buffer, 1.5 mM MgSO₄, 0.2 mM dNTPs (dATP, dUTP, dCTP, dGTP) containing dUTP instead of dTTP, 15 pmol of primers represented by SEQ ID NOs: 5 and 6 for amplifying about 1.3 kb, 10 ng of human genomic DNA (manufactured by Roche), and 1U KOD -Plus- by using the 10× PCR Buffer attached to KOD -Plus- Ver.2 (manufactured by Toyobo Co., Ltd.). Then, PCR is performed according to a schedule consisting of a pre-reaction at 94°C for 30 seconds, followed by 35 cycles of 98°C for 10 seconds, 65°C for 30 seconds, and 68°C for 1 minute and 30 seconds. After the reaction is complete, 5 µl of the reaction solution is subjected to agarose electrophoresis and then stained with ethidium bromide, followed by comparing the amplified DNA fragment of about 1.3 kb with a sample containing a wild-type PCNA or with a sample without a PCNA under UV irradiation to evaluate whether it is a PCNA capable of loading onto DNA independently. PCNAs capable of loading onto DNA independently increase the amount of amplification when added.

### (4) Amplification Bias

In the present invention, "amplification bias" refers to the non-uniform amplification of regions in multiplex PCR. The evaluation of amplification bias can be performed by confirming the uniformity of amplification in multiplex PCR, but is not limited to this method. Examples of methods for confirming the uniformity of amplification in multiplex PCR include a method of comparing band intensities through electrophoresis and a method of analyzing sequence information by subjecting amplification products to next-generation sequencing (NGS). An example of methods for analyzing GC bias in sequence information is a method using an analysis tool called "Picard." In this method, a graph is created with the GC content on the horizontal axis and the normalized coverage on the vertical axis. The graph indicates that the closer the vertical axis is to 1, the less amplification bias there is. When the vertical axis approaches 1 in the range with a GC content of about 40% to 90%, in particular 80% to 90%, it indicates that an ideal reduction in amplification bias has been achieved. The graph may also be in a trapezoidal shape.

In multiplex PCR performed according to the present invention, while the GC content in the nucleic acid region to be amplified is not particularly limited, the GC content may be 80% or higher, 85% or higher, 50 to 90%, 60 to 90%, 60 to 80%, 80 to 90%, etc.

### (5) DNA Polymerase

The DNA polymerase used in the methods and other aspects of the present invention is not particularly limited as long as it is suitable for use in PCR; the DNA polymerase preferably belongs to family B. The DNA polymerase belonging to family B is preferably derived from Archaea.

### (6) DNA Polymerase Derived from Archaea

DNA polymerases derived from Archaea belonging to family B include those derived from bacteria of the genus *Pyrococcus* and the genus *Thermococcus.*

DNA polymerases derived from the genus *Pyrococcus* include, but are not limited to, DNA polymerases isolated from *Pyrococcus furiosus, Pyrococcus sp.* GB-D, *Pyrococcus woesei, Pyrococcus abyssi,* and *Pyrococcus horikoshii.*

DNA polymerases derived from the genus *Thermococcus* include, but are not limited to, DNA polymerases isolated from *Thermococcus kodakaraensis, Thermococcus gorgonarius, Thermococcus litoralis, Thermococcus sp.* JDF-3, *Thermococcus sp.* 9 degrees North-7 *(Thermococcus sp.* 9°N-7), *Thermococcus sp.* KS-*1, Thermococcus celer,* and *Thermococcus siculi.*

PCR enzymes using these DNA polymerases are commercially available, including Pfu (Stratagene), KOD (Toyobo Co., Ltd.), Pfx (Life Technologies Corporation), Vent (New England Biolabs), Deep Vent (New England Biolabs), Tgo (Roche), Pwo (Roche), and PrimeSTAR (TaKaRa).

Of these, from the viewpoint of PCR efficiency, KOD DNA polymerase is preferable due to its excellent extensibility and thermal stability.

The DNA polymerase used in the methods and other aspects of the present invention may be a mutated form of the DNA polymerases described above and may be a mutant with mutations in the 3'-5' exonuclease region and/or mutations resulting in reduced base analog detection activity, without particular limitation.

### (7) Method for Reducing Amplification Bias

In the methods and other aspects of the present invention, as long as a PCNA is contained in a reaction solution or reagent, other components usable in multiplex PCR, such as DNA polymerase, preferably DNA polymerase belonging to family B, may also be contained in the reaction solution or reagent; however, such other components are not limited to this case.

In the methods and other aspects of the present invention, typical conditions for multiplex PCR are shown below, but are not limited to these:
(a) a DNA polymerase, preferably a DNA polymerase belonging to family B;
(b) a pair of primers in which one primer is complementary to the DNA extension product of the other primer;
(c) a DNA synthesis substrate (deoxynucleotide triphosphate (dNTP));
(d) a buffer solution containing magnesium ions, ammonium ions, and/or potassium ions; and
(e) a PCNA.

The components (a), (b), (c), (d), and (e) above are mixed, and the temperature of the reaction solution is raised and lowered using a thermal cycler or similar device to repeat the thermal cycle of (1) DNA denaturation, (2) primer annealing, and (3) primer extension, thereby amplifying a specific DNA fragment. In the PCR method above, PCR enhancing factors, BSA, non-ionic surfactants, and the like may also optionally be used.

In the PCR method above, an antibody having activity to inhibit the polymerase activity and/or 3'-5' exonuclease activity of heat-resistant DNA polymerases may also optionally be used. Examples of such antibodies include monoclonal antibodies and polyclonal antibodies. This reaction composition is particularly effective in increasing PCR sensitivity and reducing non-specific amplification.

A characteristic of the present invention is the ability to reduce amplification bias by using a PCNA in multiplex PCR. Thus, the present invention can encompass a method for improving amplification bias in multiplex PCR for preparation of a library used in next-generation sequencing, comprising the step of amplifying nucleic acids in a reaction solution containing a proliferating cell nuclear antigen. The above description of the method for reducing amplification bias of the present invention is also applicable to this method.

Furthermore, the present invention can encompass a method for preparing a library for use in next-generation sequencing, comprising the step of performing multiplex PCR in a reaction solution containing a proliferating cell nuclear antigen. The above description of the method for reducing amplification bias of the present invention is also applicable to this method. Additionally, the present invention can encompass a method for improving the amplification of target nucleic acids having a GC content of 80% or higher in multiplex PCR, comprising the step of amplifying nucleic acids in a reaction solution containing a proliferating cell nuclear antigen. The above description of the method for reducing amplification bias in the present invention is also applicable to this method. "Improvement" as used here can indicate that the amplification amount of target nucleic acids in multiplex PCR using a PCNA is greater than the amplification amount of target nucleic acids in multiplex PCR without using a PCNA.

The present invention can encompass multiplex PCR comprising the step of amplifying nucleic acids having a GC content of 80% or higher in a reaction solution containing a proliferating cell nuclear antigen. The above description of the method for reducing amplification bias of the present invention is also applicable to this multiplex PCR.

### (8) Reagent and Kit for Performing the Methods and Other Aspects of the Present Invention

The reagent and kit for performing the methods and other aspects of the present invention comprise a proliferating cell nuclear antigen, preferably a proliferating cell nuclear antigen that loads onto DNA independently, with no particular limitations on other elements. The reagent and kit of the present invention may include a DNA polymerase, preferably a DNA polymerase belonging to family B, dNTPs, primers, and other additives known in PCR reactions. The above description of the method for reducing amplification bias of the present invention is also applicable to the reagent and kit.

The reagent and kit of the present invention may include, but are not limited to, for example, a configuration comprising the following (a) to (e):
(a) an archaeal DNA polymerase mutant with reduced base analog detection activity;
(b) a pair of primers in which one primer is complementary to the DNA extension product of the other primer;
(c) a DNA synthesis substrate (deoxynucleotide triphosphate (dNTP));
(d) a buffer solution containing magnesium ions, ammonium ions, and/or potassium ions; and
(e) a PCNA.

The reagent and kit may further optionally include other reagents, such as BSA and non-ionic surfactants.

### Examples

### Example 1

### Preparation of KOD-PCNA Mutant

A plasmid containing a mutant heat-resistant PCNA gene derived from *Thermococcus kodakaraensis* KOD1 strain was constructed.

The DNA template used for mutagenesis was a PCNA derived from *Thermococcus kodakaraensis* KOD1 strain cloned into pBluescript (SEQ ID NO: 3) (pKODPCNA). Mutagenesis was performed by using a KOD -Plus- Mutagenesis Kit (manufactured by Toyobo Co., Ltd.) according to the method described in the instruction manual. The mutants were confirmed by sequencing the base sequences. *Escherichia coli* DH5α was transformed with the obtained plasmid and used for enzyme preparation.

### Example 2

### Production of Pfu-PCNA Mutant

A plasmid containing a mutant heat-resistant PCNA gene derived from *Pyrococcus furiosus* was constructed.

The DNA template used for mutagenesis was a PCNA derived from a *Pyrococcus furiosus* strain cloned into pBluescript (SEQ ID NO: 4) (pPfuPCNA). Mutagenesis was performed by using a KOD -Plus- Mutagenesis Kit (manufactured by Toyobo Co., Ltd.) according to the method described in the instruction manual. The mutants were confirmed by sequencing the base sequences. *Escherichia coli* DH5α was transformed with the obtained plasmid and used for enzyme preparation.

Table 1 shows the plasmids prepared in Example 1 and Example 2.

**Table 1**

| pKOD PCNA (SEQ ID NO: 1) | SEQ ID NO: 3 |
|---|---|
| pKOD PCNA M73L (SEQ ID NO: 9) | ATG at positions 217 to 219 of SEQ ID NO: 3 is substituted with CTG. (SEQ ID NO: 10) |
| pKOD PCNA M73L/D147A (SEQ ID NO: 15) | ATG at positions 217 to 219 of SEQ ID NO: 3 is substituted with CTG, and GAC at positions 439 to 441 is substituted with GCC. (SEQ ID NO: 16) |
| pKOD PCNA M73L/D147G (SEQ ID NO: 17) | ATG at positions 217 to 219 of SEQ ID NO: 3 is substituted with CTG, and GAC at positions 439 to 441 is substituted with GGT. (SEQ ID NO: 18) |
| pKOD PCNA M73L/E143R (SEQ ID NO: 11) | ATG at positions 217 to 219 of SEQ ID NO: 3 is substituted with CTG, and GAG at positions 427 to 429 is substituted with CGT. (SEQ ID NO: 12) |
| pKOD PCNA M73L/E143K (SEQ ID NO: 13) | ATG at positions 217 to 219 of SEQ ID NO: 3 is substituted with CTG, and GAG at positions 427 to 429 is substituted with AAA. (SEQ ID NO: 14) |
| pPfu PCNA (SEQ ID NO: 2) | SEQ ID NO: 4 |
| pPfu PCNA M73L (SEQ ID NO: 19) | ATG at positions 217 to 219 of SEQ ID NO: 4 is substituted with CTG. (SEQ ID NO: 20) |
| pPfu PCNA M73L/D143R (SEQ ID NO: 21) | ATG at positions 217 to 219 of SEQ ID NO: 4 is substituted with CTG, and GAT at positions 427 to 429 is substituted with CGT. (SEQ ID NO: 22) |
| pPfu PCNA M73L/D143K (SEQ ID NO: 23) | ATG at positions 217 to 219 of SEQ ID NO: 4 is substituted with CTG, and GAT at positions 427 to 429 is substituted with AAA. (SEQ ID NO: 24) |
| pPfu PCNA M73L/D147A (SEQ ID NO: 25) | ATG at positions 217 to 219 of SEQ ID NO: 4 is substituted with CTG, and GAT at positions 439 to 441 is substituted with GCC. (SEQ ID NO: 26) |
| pPfu PCNA M73L/D147G (SEQ ID NO: 27) | ATG at positions 217 to 219 of SEQ ID NO: 4 is substituted with CTG, and GAT at positions 439 to 441 is substituted with GGT. (SEQ ID NO: 28) |

### Example 3

### Production of Mutant Heat-resistant PCNA

The bacterial cells obtained in Example 1 were cultured as follows. First, 80 mL of sterilized TB medium (Molecular Cloning, 2nd edition, p.A.2) supplemented with 100 µg/mL of ampicillin was aliquoted into a 500 mL Sakaguchi flask. The medium was then inoculated with *Escherichia coli* DH5α (plasmidtransformed strain) (using a test tube), which had been cultured beforehand at 37°C for 16 hours in 3 mL of LB medium (1% Bacto tryptone, 0.5% yeast extract, 0.5% sodium chloride; manufactured by Gibco) supplemented with 100 µg/mL of ampicillin, and subjected to aerobic culture at 37°C for 16 hours. The bacterial cells were recovered from the culture fluid by centrifugation, suspended in 50 mL of lysis buffer (30 mM Tris-HCl buffer (pH: 8.0), 30 mM NaCl, 0.1 mM EDTA), and then lysed by sonication to obtain a cell lysate. Subsequently, the cell lysate was treated at 80°C for 15 minutes, followed by centrifugation to remove the insoluble fractions. Furthermore, the resulting cell lysate was subjected to nucleic acid removal treatment using polyethyleneimine, ammonium sulfate precipitation, and Q Sepharose chromatography, and finally, the buffer was exchanged with a storage buffer (50 mM Tris-HCl buffer (pH: 8.0), 50 mM potassium chloride, 1 mM dithiothreitol, 0.1% Tween 20, 0.1% Nonidet P-40, 50% glycerol), thereby obtaining a mutant heat-resistant PCNA.

### Example 4

### Amplification of Thermus thermophilus Library

A library with a GC content of 70% derived from *Thermus thermophilus* was amplified using various enzymes, and the post-sequencing data were compared. The library of *Thermus thermophilus* was composed of genomic fragments of *Thermus thermophilus* with a common sequence at their termini. The genome of *Thermus thermophilus* was fragmented and tagmented (adding a common sequence to the termini) using the KAPA HyperPlus Kit (KAPA Biosystems) and KAPA Unique Dual-Indexed Adapter Kit (KAPA Biosystems) and then purified using AMPure (Beckman Coulter, Inc.) to prepare the library. In the amplification of the library, KOD -Plus- (Toyobo Co., Ltd.) was used for the evaluation of KOD polymerase, and PrimeSTAR HS DNA Polymerase (TaKaRa Bio Inc.) was used for the evaluation of PrimeSTAR polymerase. The primers used for amplification were designed to hybridize with the common sequence added to the termini of the library, enabling multiplex PCR. For PCNA, the mutant heat-resistant PCNA prepared in Example 3 was used. Table 2 shows the preparation of the reaction solutions.

**Table 2**

| | KOD | KOD + PCNA1 | KOD+PCNA2 |
|---|---|---|---|
| 10 × PCR Buffer | 5 | 5 | 5 |
| 2 mM dNTPs | 5 | 5 | 5 |
| 25 mM MgSO4 | 3 | 3 | 3 |
| 10 µM Fwd Primer (SEQ ID: 7) | 5 | 5 | 5 |
| 10 µM Rev Primer (SEQ ID: 8) | 5 | 5 | 5 |
| 0.25 ng/µL Library | 4 | 4 | 4 |
| KOD - Plus - | 1 | 1 | 1 |
| 250 ng/µL KOD-PCNA D147A | - | 1 | - |
| 250 ng/µL Pfu-PCNA E143R | - | - | 1 |
| H2O | 22 | 21 | 21 |

| | PS | PS + PCNA2 |
|---|---|---|
| 5 ×PrimeSTAR Buffer (Mg2+plus) | 10 | 10 |
| dNTP Mixture | 4 | 4 |
| PrimeSTAR HS DNA Polymerase | 1 | 1 |
| 10 µM Fwd Primer (SEQ ID: 7) | 5 | 5 |
| 10 µM Rev Primer (SEQ ID: 8) | 5 | 5 |
| 0.25 ng/µL Library | 4 | 4 |
| 250 ng/µL Pfu-PCNA E143R | - | 1 |
| H2O | 21 | 20 |

Amplification was performed in the following manner: 72°C for 3 minutes, 95°C for 30 seconds, followed by 14 cycles of 95°C for 10 seconds, 60°C for 30 seconds, and 72°C for 30 seconds. The amplification products purified using AMPure were sequenced using an Illumina MiSeq System. The post-sequencing data were normalized to 200,000 reads and evaluated using Picard (CollectGcBiasMetrics) and FastQC.

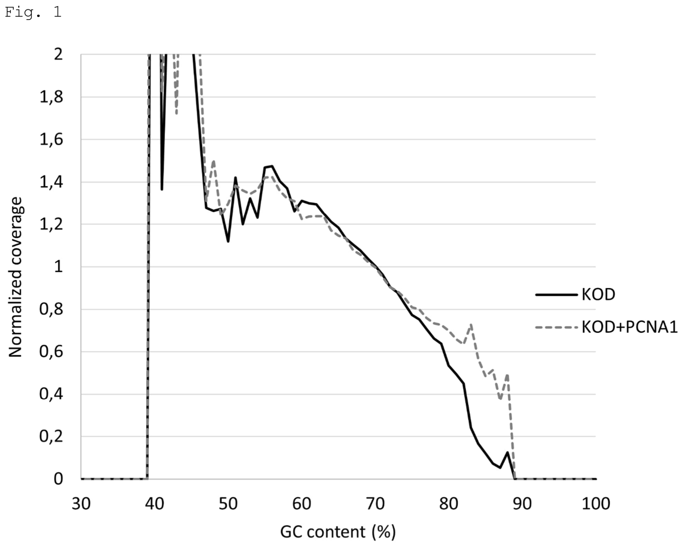

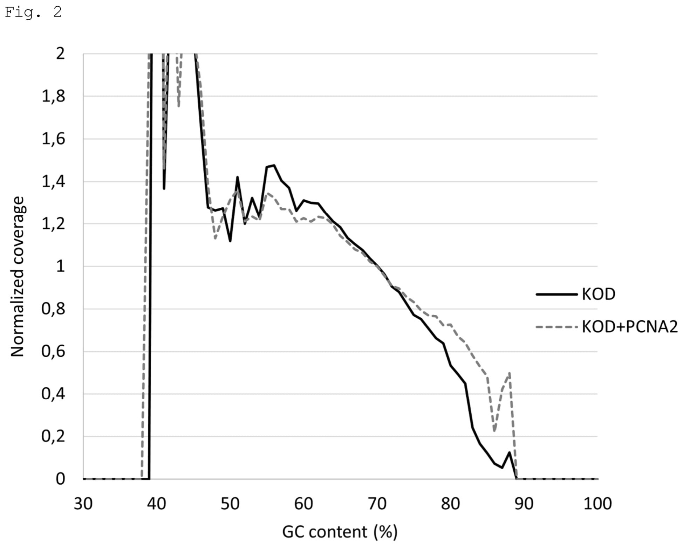

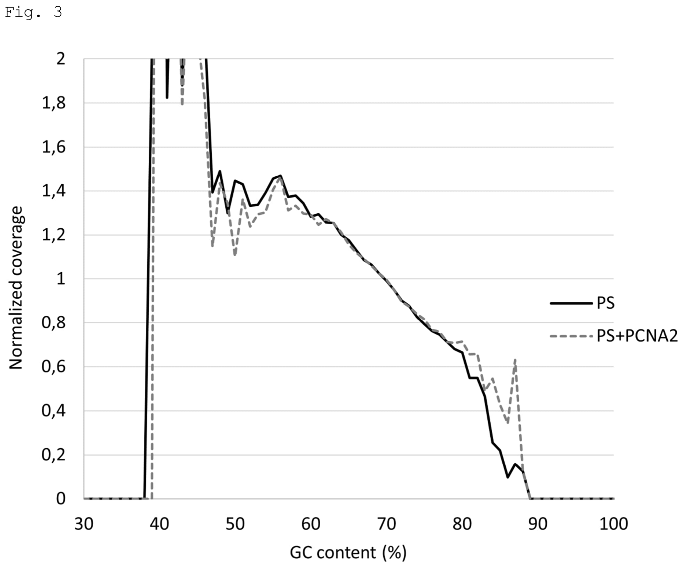

Figs. 1 to 3 show the evaluation results of GC bias using Picard (normalized coverage against GC content). In the figures, the horizontal axis represents the GC content, while the vertical axis represents the normalized coverage. The closer the normalized coverage is to 1, the more the bias has been reduced. PCNA1 (KOD-PCNA M73L/D147A) and PCNA2 (Pfu-PCNA M73L/D143R) respectively were added to the KOD reaction system in Fig. 1 and the KOD reaction system in Fig. 2. While the normalized coverage deteriorated significantly when the GC content exceeded 80% without PCNA addition, the addition of a PCNA resulted in an improvement in this deterioration. Fig. 3 shows the results of adding PCNA2 (Pfu-PCNA M73L/D143R) to the PrimeSTAR (PS) reaction system in the same manner. For PrimeSTAR as well, an improvement in normalized coverage was confirmed at high GC contents due to the addition of a PCNA.

In the same manner, KOD-PCNA M73L/D147G, KOD-PCNA M73L/E143R, KOD-PCNA M73L/E143K, Pfu-PCNA M73L/D143K, Pfu-PCNA M73L/D147A, and Pfu-PCNA M73L/D147G were added to the KOD reaction system; and KOD-PCNA M73L/D147A, KOD-PCNA M73L/D147G, KOD-PCNA M73L/E143R, KOD-PCNA M73L/E143K, Pfu-PCNA M73L/D143K, Pfu-PCNA M73L/D147A, and Pfu-PCNA M73L/D147G were added to the PrimeSTAR (PS) reaction system. In both cases, improvements in normalized coverage were confirmed.

Table 3 shows the coverage rate in the *Thermus thermophilus* genome for sequence depths of 1 to 5 as analyzed by FastQC. X1 to X5 represent the depth (the number of times the *Thermus thermophilus* genome was sequenced), and the coverage rate indicates the proportion of sequenced regions relative to the total length of the *Thermus thermophilus* genome. For example, X5 indicates the proportion of the region with 5 or more sequence reads relative to the total genome length, and a larger proportion indicates a greater sequenced region.

**Table 3**

| | KOD | KOD+PCNA1 | KOD+PCNA2 | PS | PS+PCNA2 |
|---|---|---|---|---|---|
| X1 | 99.8 | 100.0 | 100.0 | 99.9 | 100.0 |
| X2 | 99.5 | 99.9 | 99.9 | 99.7 | 99.8 |
| X3 | 98.8 | 99.4 | 99.5 | 99.2 | 99.4 |
| X4 | 97.4 | 98.4 | 98.5 | 98.0 | 98.3 |
| X5 | 95.1 | 96.4 | 96.6 | 95.9 | 96.2 |

The coverage rates in the case with addition of a PCNA were better. In this Example, the test was conducted with a relatively small number of reads: 200,000; it is believed that the addition of a PCNA reduced amplification bias and improved coverage rate. The amount of sequencing (number of reads) is directly linked to cost. Adding a PCNA achieves a higher coverage rate with the same number of reads, thus contributing to cost reduction.

### Industrial Applicability

The present invention provides a method for reducing amplification bias in multiplex PCR and other related aspects. The present invention enables the reduction of amplification bias, which is expected to minimize oversight in multiplex PCR and improve accuracy. The reduced amplification bias due to the present invention also leads to improved sequencing accuracy in NGS, thus achieving high coverage rates even with fewer sequence reads and contributing to cost reduction.

### Sequence Listing

P24-082WO_PCT_Amplification_in_nucleic_acid_amplification_20240620_110732_3.xml

## Claims

1. A method for reducing amplification bias in multiplex PCR, comprising the step of amplifying nucleic acids in a reaction solution containing a proliferating cell nuclear antigen.

2. The method according to claim 1, wherein the proliferating cell nuclear antigen comprises
(a) a polypeptide containing an amino acid sequence in which at least one amino acid selected from the group consisting of amino acids at positions 82, 84, and 109 in the amino acid sequence of SEQ ID NO: 1 or 2 is mutated, or
(b) a polypeptide containing an amino acid sequence in which at least one amino acid selected from the group consisting of amino acids at positions 139, 143, and 147 in the amino acid sequence of SEQ ID NO: 1 or 2 is mutated, or
(c) a polypeptide containing an amino acid sequence in which at least one amino acid selected from the group consisting of amino acids at positions 82, 84, and 109 in the amino acid sequence of SEQ ID NO: 1 or 2, and at least one amino acid selected from the group consisting of amino acids at positions 139, 143, and 147 in the amino acid sequence of SEQ ID NO: 1 or 2 are mutated, or
(d) a polypeptide containing an amino acid sequence in which one to several amino acids are mutated among amino acids other than amino acids corresponding to positions 82, 84, 109, 139, 143, and 147 in the amino acid sequence of SEQ ID NO: 1 or 2 in any of the amino acid sequences contained in the polypeptides (a) to (c), or
(e) a polypeptide containing an amino acid sequence having at least 80% identity with any of the amino acid sequences contained in the polypeptides (a) to (c), wherein at least one amino acid corresponding to a position selected from the group consisting of positions 82, 84, 109, 139, 143, and 147 in the amino acid sequence of SEQ ID NO: 1 or 2 is mutated.

3. The method according to claim 2,
wherein
the amino acid corresponding to position 143 is a basic amino acid,
the amino acid corresponding to position 147 is a neutral amino acid, or
the amino acid corresponding to position 143 is a basic amino acid and the amino acid corresponding to position 147 is a neutral amino acid.

4. The method according to claim 2,
wherein
the amino acid corresponding to position 143 is arginine or lysine,
the amino acid corresponding to position 147 is alanine or glycine, or
the amino acid corresponding to position 143 is arginine or lysine and the amino acid corresponding to position 147 is alanine or glycine.

5. The method according to claim 2, wherein in the amino acid sequence contained in any of the polypeptides (a) to (e), the amino acid corresponding to position 73 in the amino acid sequence of SEQ ID NO: 1 or 2 is leucine.

6. The method according to claim 2, wherein the polypeptide (d) contains an amino acid sequence in which one to three amino acids are mutated among amino acids other than amino acids corresponding to positions 82, 84, 109, 139, 143, and 147 in the amino acid sequence of SEQ ID NO: 1 or 2 in any of the amino acid sequences contained in the polypeptides (a) to (c).

7. The method according to claim 2, wherein the mutated means that an amino acid is substituted, deleted, inserted, and/or added.

8. The method according to claim 2, wherein the polypeptide (e) contains an amino acid sequence having at least 90% identity with any of the amino acid sequences contained in the polypeptides (a) to (c), wherein at least one amino acid corresponding to a position selected from the group consisting of positions 82, 84, 109, 139, 143, and 147 in the amino acid sequence of SEQ ID NO: 1 or 2 is mutated.

9. The method according to claim 1 or 2, wherein at least one nucleic acid amplified in the multiplex PCR has a GC content of 80% or higher.

10. The method according to claim 1, wherein the reaction solution further contains a DNA polymerase belonging to family B.

11. The method according to claim 10, wherein the DNA polymerase belonging to family B is derived from Archaea.

12. The method according to claim 10, wherein the DNA polymerase belonging to family B is derived from bacteria of the genus *Pyrococcus* or the genus *Thermococcus.*

13. A method for improving amplification bias in multiplex PCR for preparation of a library used in next-generation sequencing, comprising the step of amplifying nucleic acids in a reaction solution containing a proliferating cell nuclear antigen.

14. A method for preparing a library for use in next-generation sequencing, comprising the step of performing multiplex PCR in a reaction solution containing a proliferating cell nuclear antigen.

15. A method for improving amplification of a target nucleic acid having a GC content of 80% or higher in multiplex PCR, comprising the step of amplifying nucleic acids in a reaction solution containing a proliferating cell nuclear antigen.

16. A multiplex PCR comprising the step of amplifying a nucleic acid having a GC content of 80% or higher in a reaction solution containing a proliferating cell nuclear antigen.

17. A reagent for performing the method of any one of claims 1 and 13 to 15 or performing the multiplex PCR of claim 16, comprising a proliferating cell nuclear antigen containing
(a) a polypeptide containing an amino acid sequence in which at least one amino acid selected from the group consisting of amino acids at positions 82, 84, and 109 in the amino acid sequence of SEQ ID NO: 1 or 2 is mutated, or
(b) a polypeptide containing an amino acid sequence in which at least one amino acid selected from the group consisting of amino acids at positions 139, 143, and 147 in the amino acid sequence of SEQ ID NO: 1 or 2 is mutated, or
(c) a polypeptide containing an amino acid sequence in which at least one amino acid selected from the group consisting of amino acids at positions 82, 84, and 109 in the amino acid sequence of SEQ ID NO: 1 or 2, and at least one amino acid selected from the group consisting of amino acids at positions 139, 143, and 147 in the amino acid sequence of SEQ ID NO: 1 or 2 are mutated, or
(d) a polypeptide containing an amino acid sequence in which one to several amino acids are mutated among amino acids other than amino acids corresponding to positions 82, 84, 109, 139, 143, and 147 in the amino acid sequence of SEQ ID NO: 1 or 2 in any of the amino acid sequences contained in the polypeptides (a) to (c), or
(e) a polypeptide containing an amino acid sequence having at least 80% identity with any of the amino acid sequences contained in the polypeptides (a) to (c), wherein at least one amino acid corresponding to a position selected from the group consisting of positions 82, 84, 109, 139, 143, and 147 in the amino acid sequence of SEQ ID NO: 1 or 2 is mutated.

18. A kit for performing the method of any one of claims 1 and 13 to 15 or performing the multiplex PCR of claim 16, comprising the reagent of claim 17.
